# EUROPEAN PATENT APPLICATION

(11) **EP 4 707 312 A1**
(43) Date of publication of application: **11.03.2026**
(21) Application number: 24909208.1
(22) Date of filing: 01.08.2024
(51) Int. Cl.: C08F 212/08

(54) **RNA ROOM-TEMPERATURE PRESERVATION STABILIZER, RNA PRODUCT, PREPARATION METHOD, AND USE**

(30) Priority: 15.07.2024 CN 202410942297
(71) Applicant: Bisheng (Beijing) Biotechnology Co., Ltd., Beijing (CN)
(72) Inventor: WANG, Daming, Beijing 100083 (CN); CAO, Qinghao, Beijing 100083 (CN); CAO, Yuhong, Beijing 100083 (CN)
(74) Representative: Murgitroyd & Company
(86) International application number: PCT/CN2024/109183
(87) International publication number: WO 2026/016223

(57) **Abstract**

An RNA room-temperature storage stabilizer, an RNA product, and a preparation method and use thereof are disclosed, belonging to the field of biotechnology. The RNA room-temperature storage stabilizer provided by the present disclosure includes a functionalized polystyrene microsphere. The RNA room-temperature storage stabilizer has good adsorption capacity for RNA. The functional groups on the RNA room-temperature storage stabilizer can interact with the groups in the RNA, which plays a role in stabilizing the structure of the RNA, thereby realizing long-term stable storage of the RNA at room temperature without the need of introducing an ultra-low temperature device and other protective reagents; and the addition of water can destroy the binding between the functionalized polystyrene microsphere and the RNA, so as to achieve complete separation of the RNA from the stabilizer. It has the advantages of low cost, simple operation and high biosafety.

## Description

### TECHNICAL FIELD

The present disclosure belongs to the field of biotechnology, and in particular relates to an RNA room-temperature storage stabilizer, an RNA product, and a preparation method and use thereof.

### BACKGROUND

RNAs are long chain molecules formed by condensation of ribonucleotides through phosphodiester bonds. They are widely present in biological cells, some viruses and viroids. Among the RNAs, mRNA can be responsible for transmitting genetic information during protein synthesis and directly guiding protein synthesis. The mRNA can be applied to vaccines (infectious vaccines and tumor vaccines), protein replacement therapies, cell engineering, gene editing and the like fields - by transferring the mRNAs into the cells of a patient (or recipient), so that the mRNA can be successfully translated into specified proteins, and trigger effective humoral and cellular immune responses to achieve the purpose of preventing diseases or changing specific disease states. Compared with traditional drugs and vaccines, mRNA vaccines can be designed to encode any antigen according to the unique attributes of a disease and regulate the expression of the selected antigen, thereby being able to quickly respond to mutations in pathogenic microorganisms and provide more timely and reliable protection. However, the structural stability defects of the mRNA themselves make them inevitable to degrade during storage and transportation, resulting in structural changes and reduced quality, which in turn affects the biological activity of the mRNA.

Currently, the mRNA is generally stored by cryopreservation, external addition of protective agents, or a combination of the two. However, during the cryopreservation, the mRNA is generally stored in an ultra-low temperature refrigerator or even liquid nitrogen at -80°C to minimize their degradation rate. This places high requirements on transportation and application scenarios, and will lead to increased energy consumption, increased transportation costs, and safety issues. The introduction of the protective agents may reduce the biosafety of the mRNA drugs or vaccines, and has great limitations.

### SUMMARY

A first objective of the present disclosure is to provide an RNA room-temperature storage stabilizer, for solving the problems of high requirements on storage conditions, high storage costs and low biosafety etc. existed in existing mRNA storage, and obtaining a simple, effective, economical and practical method with high biosafety to achieve long-term stable storage and transportation of mRNA.

A second objective of the present disclosure is to provide a method for preparing the aforementioned RNA room-temperature storage stabilizer.

A third objective of the present disclosure is to provide an RNA product.

A fourth objective of the present disclosure is to provide a method for preparing an RNA product.

A fifth objective of the present disclosure is to provide use of the aforementioned RNA room-temperature storage stabilizer and/or method for preparing an RNA product in production of a mRNA drug and/or vaccine.

Specifically, the RNA room-temperature storage stabilizer provided by the present disclosure includes a functionalized polystyrene microsphere.

In some specific embodiments, the functionalized polystyrene microsphere is a polystyrene microsphere modified with a functional group, and the functional group is selected from one or more of C1-C20 chloroalkyl, C6-C20 chlorophenyl, C1-C20 hydroxyl-substituted alkyl, C6-C20 hydroxyl-substituted phenyl, C2-C20 carboxyl-substituted alkyl, C7-C20 carboxyl-substituted phenyl, C1-C20 amino-substituted alkyl, C6-C20 amino-substituted phenyl, and C4-C20 nitrogen-containing heterocycle.

In some specific embodiments, the functionalized polystyrene microsphere is selected from one or more of a chloromethyl modified polystyrene microsphere, a hydroxyl modified polystyrene microsphere, a carboxyl modified polystyrene microsphere, and a nitrogen-containing five-membered heterocyclic modified polystyrene microsphere.

In some specific embodiments, an average particle size of the functionalized polystyrene microsphere is 0.1-200 µm.

In some specific embodiments, an average particle size of the functionalized polystyrene microsphere is 1-2 µm.

The method for preparing the aforementioned RNA room-temperature storage stabilizer as provided by the present disclosure includes: S1. taking a polymerizable monomer, a functional modifier, an initiator and an optional polystyrene particle to conduct a polymerization reaction to obtain the functionalized polystyrene microsphere; and S2. taking and subjecting the functionalized polystyrene microsphere to denuclease treatment to obtain the RNA room-temperature storage stabilizer.

In some specific embodiments, in the step S1, the polymerizable monomer is selected from one or more of styrene, phenylpropene and divinylbenzene.

In some specific embodiments, in the step S1, the functional modifier is selected from one or more of a chlorinated olefin, an enol, an unsaturated fatty acid and an olefinized pyrrolidone.

In some specific embodiments, in the step S1, the initiator is an azo initiator and/or a peroxy initiator.

In some specific embodiments, in the step S1, the average particle size of the polystyrene particle is 0.01-1 µm.

In some specific embodiments, in the step S1, an input mass ratio of the polymerizable monomer, the functional modifier, the initiator and the polystyrene particle is (5-20):(0.1-20):(0.1-10):(0.001-20).

In some specific embodiments, in the step S1, the polymerization reaction is conducted at a temperature of 70-90°C for a time of 1-48 h.

In some specific embodiments, the denuclease treatment specifically includes: taking and mixing the functionalized polystyrene microsphere with RNase-free water, and then subjecting to centrifugal column purification to obtain the RNA room-temperature storage stabilizer.

In some specific embodiments, the centrifugal column purification is conducted at a centrifugal speed of 5,000-1,0000 rpm for a time of 5-30 min.

The RNA product provided by the present disclosure includes the aforementioned RNA room-temperature storage stabilizer.

The method for preparing the RNA product as provided by the present disclosure includes: taking and mixing an RNA with the RNA room-temperature storage stabilizer, and then subjecting to freeze-drying treatment to obtain the RNA product.

In some specific embodiments, an input mass ratio of the RNA to the RNA room-temperature storage stabilizer is 1:(10-1000).

In some specific embodiments, the freeze-drying treatment includes a pre-freezing stage, a primary drying stage and a secondary drying stage, the pre-freezing stage is conducted at a temperature of -60 - -50°C for a time of 5-15 h; the primary drying stage is conducted at a temperature of -45 - -40°C with a vacuum degree no higher than 10 Pa for a time of 1-5 h; the secondary drying stage includes a heating period and a constant temperature period that are conducted alternately, the secondary drying stage is conducted at a starting temperature of -35 - -30°C and a final temperature of 20-25°C with a vacuum degree no higher than 10 Pa for a total time of 40-60 h, and the heating period is conducted at a heating rate of 0.5-2°C/min for a time of 25-40 min.

The present disclosure further provides use of the aforementioned RNA room-temperature storage stabilizer, RNA product and/or method for preparing the RNA product in production of a mRNA drug and/or vaccine.

### Beneficial effects:

The present disclosure provides an RNA room-temperature storage stabilizer including a functionalized polystyrene microsphere. The functionalized polystyrene microsphere has good adsorption capacity for RNA. The functional groups on the microsphere can interact with the groups in the RNA, so that the RNA molecules rotate, twist, entangle and fold to form a specific three-dimensional structure, which plays a role in stabilizing the structure of the RNA, thereby realizing long-term stable storage of the RNA at room temperature without the need of introducing an ultra-low temperature device and other protective reagents; and the addition of water can destroy the binding between the functionalized polystyrene microsphere and the RNA, so as to achieve complete separation of the RNA from the stabilizer. It has the advantages of low cost, simple operation and high biosafety, and has good application prospects.

### BRIEF DESCRIPTION OF THE DRAWINGS

FIG. 1 is an infrared spectrogram of a PS-Cl microsphere prepared in Preparation Example 1 of the present disclosure;
FIG. 2 is a SEM image of the PS-Cl microsphere prepared in Preparation Example 1 of the present disclosure (200 µm);
FIG. 3 is an infrared spectrogram of a PS-OH microsphere prepared in Preparation Example 2 of the present disclosure;
FIG. 4 is a SEM image of the PS-OH microsphere prepared in Preparation Example 2 of the present disclosure (5.0 µm);
FIG. 5 is an infrared spectrogram of a PS-COOH microsphere prepared in Preparation Example 3 of the present disclosure;
FIG. 6 is a SEM image of the PS-COOH microsphere prepared in Preparation Example 3 of the present disclosure (5.0 µm);
FIG. 7 is an infrared spectrogram of a PS-N microsphere prepared in Preparation Example 4 of the present disclosure;
FIG. 8 is a SEM image of the PS-N microsphere prepared in Preparation Example 4 of the present disclosure (5.0 µm);
FIG. 9 is a diagram showing an experimental result of an electrophoresis test of an RNA product stored at 25°C for 7 d in a Test Example of the present disclosure;
FIG. 10 is a diagram showing an experimental result of an electrophoresis test of an RNA product stored at 25°C for 21 d in a Test Example of the present disclosure;
FIG. 11 is a diagram showing an experimental result of an electrophoresis test of an RNA product stored at 37°C for 7 d in a Test Example of the present disclosure;
FIG. 12 is a diagram showing an experimental result of an electrophoresis test of an RNA product stored at 37°C for 21 d in a Test Example of the present disclosure;
FIG. 13 is a diagram showing an experimental result of an electrophoresis test of an RNA product stored at 60°C for 1 d in a Test Example of the present disclosure;
FIG. 14 is a diagram showing an experimental result of an electrophoresis test of an RNA product stored at 60°C for 5 d in a Test Example of the present disclosure; and
FIG. 15 is a diagram showing an experimental result of an electrophoresis test of an RNA product stored at 60°C for 14 d in a Test Example of the present disclosure.

### DETAILED DESCRIPTION OF THE EMBODIMENTS

The RNA room-temperature storage stabilizer provided by the present disclosure specifically includes a functionalized polystyrene microsphere. The functionalized polystyrene microsphere is specifically a polystyrene microsphere modified with a functional group, and specific examples of the functional group include one or more of C1-C20 chloroalkyl, C6-C20 chlorophenyl, C1-C20 hydroxyl-substituted alkyl, C6-C20 hydroxyl-substituted phenyl, C2-C20 carboxyl-substituted alkyl, C7-C20 carboxyl-substituted phenyl, C1-C20 amino-substituted alkyl, C6-C20 amino-substituted phenyl, and C4-C20 nitrogen-containing heterocycle. Specific examples of the C1-C20 chloroalkyl include, but are not limited to: -CH₂Cl, -CH₂CH(Cl)CH₃ or -(CH₂)₅CH(Cl)CH₃. Specific examples of the C6-C20 chlorophenyl include, but are not limited to: 1-chloromethylphenyl, 2-chloromethylphenyl or 3-chloroethylphenyl. Specific examples of the C1-C20 hydroxy-substituted alkyl include, but are not limited to: -CH₂OH, -CH₂CH₂OH, -CH₂CH(OH)CH₃ or -(CH₂)₅CH(OH)CH₃. Specific examples of the C6-C20 hydroxy-substituted phenyl include, but are not limited to: 1-hydroxyphenyl, 2-hydroxyphenyl or 3-hydroxyphenyl. Specific examples of the C2-C20 carboxyl-substituted alkyl include, but are not limited to: -CH₂COOH, -(CH₂)₁₂COOH or -(CH₂)₈CH(COOH)CH₃. Specific examples of the C7-C20 carboxyl-substituted phenyl include, but are not limited to: 1-carboxyphenyl, 2-carboxyphenyl or 1-carboxyethylphenyl. Specific examples of the C1-C20 amino-substituted alkyl include, but are not limited to: -CH₂NH₃, -CH₂NH₃, -CH₂CH₂NH₃, -CH₂CH(NH₃)CH₃ or -(CH₂)₅CH(NH₃)CH₃. Specific examples of the C6-C20 amino-substituted phenyl include, but are not limited to: 1-aminophenyl, 2-aminophenyl or 1-aminomethylphenyl. The C4-C20 nitrogen-containing heterocycle is preferably a nitrogen-containing five-membered heterocycle, and more preferably an N-ethyl-2-pyrrolidone group or a 1-(propyl)pyrrolidin-2-one group.

In the present disclosure, the functionalized polystyrene microsphere is preferably one or more of a chloromethyl modified polystyrene microsphere, a hydroxyl modified polystyrene microsphere, a carboxyl modified polystyrene microsphere, and a nitrogen-containing five-membered heterocyclic modified polystyrene microsphere. In some specific embodiments, the functionalized polystyrene microsphere is more preferably a nitrogen-containing five-membered heterocyclic modified polystyrene microsphere. At this time, the nitrogen-containing five-membered heterocycle modified polystyrene microsphere is rich in functional groups which has a five-membered heterocyclic structure similar to ribose, which not only has a more ideal effect of stabilizing the structure of the RNA, but also can slow down the cleavage of the RNA by a ribonuclease, and has better protection and stabilization functions for the RNA.

In the present disclosure, an average particle size of the functionalized polystyrene microsphere is preferably 0.1-200 µm, e.g. 0.1 µm, 0.5 µm, 1 µm, 5 µm, 10 µm, 15.6 µm, 25 µm, 50 µm, 78 µm, 90 µm, 99 µm, 100 µm, 150 µm, 200 µm, or any value therebetween. In some specific embodiments, an average particle size of the functionalized polystyrene microsphere is more preferably 1-2 µm.

A method for preparing the aforementioned RNA room-temperature storage stabilizer as provided by the present disclosure specifically includes: S1. taking a polymerizable monomer, a functional modifier, an initiator and an optional polystyrene particle to conduct a polymerization reaction to obtain the functionalized polystyrene microsphere; and S2. taking and subjecting the functionalized polystyrene microsphere to denuclease treatment to obtain the RNA room-temperature storage stabilizer.

In the present disclosure, the functional modifier in the step S1 is a class of compounds that can react with the polymerizable monomer and in turn introduce the functional group into the polystyrene microsphere during the polymerization of polystyrene. The functional modifier can be selected according to the desired functionalized polystyrene microsphere to be obtained, and is not particularly limited. Specific examples of the functional modifier include, but are not limited to: one or more of a chlorinated olefin, an enol, an unsaturated fatty acid and an olefinized pyrrolidone. The chlorinated olefin may specifically be, but not limited to one or more of 2-chlorostyrene, 3-chlorostyrene, chloromethylstyrene, 1-chloropropene and 3-chloropropene. The enol may specifically be, but is not limited to, one or more of 2-propen-1-ol, 3-buten-1-ol, 2-hydroxystyrene and 3-hydroxystyrene. Specific examples of the unsaturated fatty acid include, but are not limited to: one or more of oleic acid, linoleic acid, linolenic acid and arachidonic acid. The olefinized pyrrolidone may specifically be, but is not limited to, N-vinyl-2-pyrrolidone and/or 1-(allyl)pyrrolidin-2-one.

In the present disclosure, the initiator in the step S1 is a class of compounds commonly used in existing polystyrene polymerization, and is not particularly limited, as long as it capable of decomposing to generate free radicals and initiate polymerization of the polymerizable monomers. Specific examples of the initiator include, but are not limited to: an azo initiator and/or a peroxy initiator. The azo initiator may specifically be, but not limited to, one or more of azobisisobutyronitrile, azobisisoheptanenitrile, dimethyl 2,2'-azobis(2-methylpropionate) and 2-(1-Cyano-1-methylethyl)azocarboxamide. The peroxide initiator may specifically be, but is not limited to, one or more of benzoyl peroxide, tert-butyl peroxy-2-ethylhexanoate and tert-butyl peroxybenzoate.

In the present disclosure, the addition of the polystyrene particle in step S1 is beneficial to the control of the shape and particle size of the microsphere, thereby obtaining a functionalized polystyrene microsphere with better storage stability for the RNA; and the particle size of the polystyrene particle is preferably 0.01-1 µm, e.g. 0.01 µm, 0.03 µm, 0.05 µm, 0.07 µm, 0.1 µm, 0.25 µm, 0.4 µm, 0.6 µm, 0.8 µm, 1 µm or any value therebetween.

In the present disclosure, the input mass ratio of the polymerizable monomer, the functional modifier, the initiator and the polystyrene particle in the step S1 is preferably (5-20):(0.1-20):(0.1-10):(0.001-20), e.g. 5:0.1:0.1:0.001, 5:13:7:19, 16:3:7:20, 20:0.1:0.1:19, 20:20:10:20 or any value therebetween.

In the present disclosure, the conditions of the polymerization reaction in the step S1 include a temperature of preferably 70-90°C, e.g. 70°C, 71.5°C, 72°C, 73°C, 75°C, 78°C, 80°C, 81°C, 84°C, 88°C, 90°C or any value therebetween; and a time of preferably 1-48 h, e.g. 1 h, 4 h, 10 h, 12 h, 15 h, 18 h, 24 h, 30 h, 38 h, 48 h or any value therebetween.

In the present disclosure, the denuclease treatment in the step S2 specifically includes: taking and mixing the functionalized polystyrene microsphere with RNase-free water, and then subjecting to centrifugal column purification to obtain the RNA room-temperature storage stabilizer.

In some specific embodiments, the conditions for the centrifugal column purification include a centrifugal speed of preferably 5,000-1,0000 rpm, e.g. 5,000 rpm, 5,100 rpm, 5,500 rpm, 6,000 rpm, 7,000 rpm, 8,000 rpm, 9,000 rpm or any value therebetween; and a time of preferably 5-30 min, e.g. 5 min, 7 min, 10 min, 13 min, 15 min, 20 min, 25 min, 27 min, 30 min or any value therebetween.

The RNA product provided by the present disclosure includes the aforementioned RNA room-temperature storage stabilizer; and by compounding the RNA room-temperature storage stabilizer with the RNA, the RNA product can be endowed with good room-temperature storage stability.

The method for preparing the aforementioned RNA product as provided by the present disclosure specifically includes: taking and mixing an RNA with an RNA room-temperature storage stabilizer, and then subjecting to freeze-drying treatment to obtain the RNA product.

In the present disclosure, the input mass ratio of the RNA to the RNA room-temperature storage stabilizer is preferably 1:(10-1000), e.g. 1:10, 1:50, 1:100, 1:125, 1:500, 1:1000 or any value therebetween.

In the present disclosure, the freeze-drying treatment may be carried out by the conventional methods and conditions used in the preparation of RNA products in the prior art, and such methods are not particularly limited, as long as they can achieve dehydration and drying.

In the present disclosure, the freeze-drying process may also be a process designed by the inventors of the present disclosure after creative work based on their profound understanding of the changes of the aforementioned RNA room-temperature storage stabilizer, the RNA and the water molecule during the treatment process, and the process specifically includes: a pre-freezing stage, a primary drying stage and a secondary drying stage. The pre-freezing stage is conducted at a temperature of preferably -60 - -50°C, e.g. -60°C, -58°C, -54°C, -52°C, -51°C, -50°C or any value therebetween, for a time of preferably 5-15 h, e.g. 5 h, 5.8 h, 6 h, 7 h, 9 h, 10 h, 12 h, 15 h or any value therebetween. The primary drying stage is conducted at a temperature of preferably -45 - -40°C, e.g. -45°C, -44.8°C, -43°C, -41.5°C, -40°C or any value therebetween, with a vacuum degree of preferably no higher than 10 Pa, more preferably 0-9 Pa, e.g. 0 Pa, 0.001 Pa, 0.1 Pa, 0.5 Pa, 1 Pa, 3 Pa, 5 Pa, 7 Pa, 9 Pa or any value therebetween, for a time of preferably 1-5 h, e.g. 1 h, 1.3 h, 1.5 h, 2 h, 2.5 h, 3 h, 4 h, 5 h or any value therebetween. The secondary drying stage includes a heating period and a constant temperature period which are conducted alternately. The secondary drying stage is conducted at a starting temperature of preferably -35 - -30°C, e.g. -35°C, -34.8°C, -33°C, -31°C, -30°C or any value therebetween and a final temperature of preferably 20 - 25°C, e.g. 20°C, 21°C, 23°C, 24°C, 25°C or any value therebetween, with a vacuum degree of preferably no higher than 10 Pa, e.g. 0 Pa, 0.001 Pa, 0.3 Pa, 1.5 Pa, 3.2 Pa, 5 Pa, 9 Pa or any value therebetween, for a total time of preferably 40-60 h, e.g. 40 h, 41 h, 43 h, 45 h, 50 h, 51 h, 56 h, 59 h, 60 h or any value therebetween; and the heating period is conducted at a heating rate of preferably 0.5-2°C/min, e.g. 0.5°C/min, 0.6°C/min, 0.8°C/min, 1°C/min, 1.5°C/min, 1.8°C/min, 2°C/min or any value therebetween, for a time of preferably 25-40 min, e.g. 25 min, 25.6 min, 28 min, 30 min, 33 min, 35 min, 37.6 min, 38 min, 40 min or any value therebetween. At this time, the use of a method of segmented freeze-drying supplemented by intermittent heating can effectively reduce the damage to the structure of the RNA caused by the freeze-drying process, and achieve a better binding effect between the functionalized polystyrene microsphere and the RNA, further improving the room-temperature storage stability of the RNA product.

The present disclosure further provides use of the aforementioned RNA room-temperature storage stabilizer, RNA product and/or method for preparing the RNA product in production of a mRNA drug and/or vaccine.

Hereinafter, embodiments of the present disclosure will be described in detail, and examples of the embodiments are intended to explain the present disclosure and should not be construed as limiting the present disclosure. If no specific technology or condition is indicated in the examples, it shall be carried out according to the technology or condition described in the literature in the art or according to product instructions. All of the used agents or instruments which are not specified with the manufacturer are conventional commercially-available products.

### Synthesis Example

This synthesis example was used for illustrating the preparation of a polystyrene microsphere, which specifically included the following steps: 9.8 mL of styrene, 15 mL of absolute ethanol and 125 mL of deionized water were accurately pipetted into a round-bottom flask, added with 652 mg of polyvinylpyrrolidone (PVP K-30, with a weight average molecular weight of 58,000) and 153.2 mg of benzoyl peroxide (BPO), subjected to vacuum degassing, and quickly stirred until it became an emulsion; then reacted in a 73°C oil bath under a nitrogen atmosphere for 6 h; and cooled to room temperature after the reaction was completed. The obtained emulsion was washed repeatedly with absolute ethanol for 10 times, then continually washed with deionized water for 8 times, and vacuum-dried at 60°C for 48 h to obtain a polystyrene microsphere, which was designated as a PS microsphere.

### Preparation Example 1

This preparation example was used for illustrating the preparation of a chloromethyl modified polystyrene microsphere, which specifically included the following steps: 17.6 mL of styrene, 2 mL of divinylbenzene, 5 mL of chloromethylstyrene and 250 mL of deionized water were accurately pipetted into a round-bottom flask, added with 2.0 g of polyvinyl alcohol (with viscosity of 6.0 mPa·s) and 500 mg of BPO, subjected to vacuum degassing and rapidly stirred until it became an emulsion; then reacted in a 82°C oil bath under a nitrogen atmosphere for 4 h; and cooled to room temperature after the reaction was completed. The obtained emulsion was repeatedly washed with absolute ethanol for 10 times, then continually washed with deionized water for 8 times, and vacuum-dried at 60°C for 48 h to obtain a chloromethyl-modified polystyrene microsphere, which was designated as a PS-Cl microsphere.

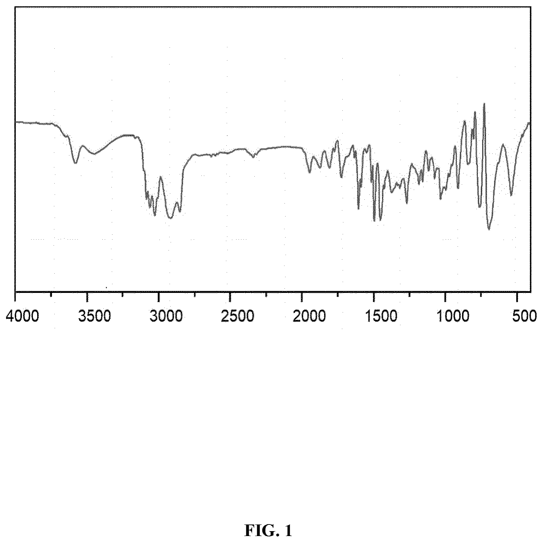

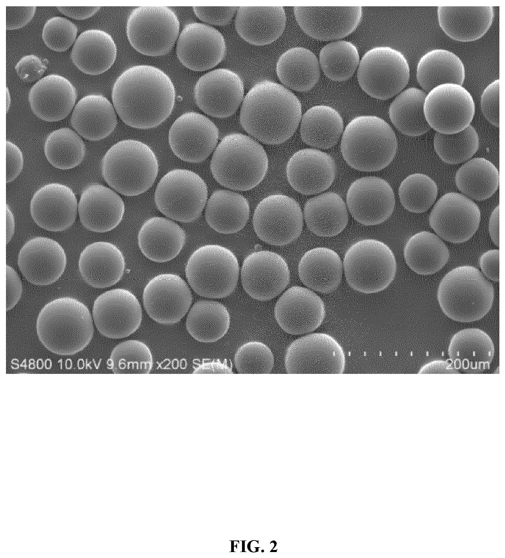

The SEM image and infrared spectrogram of the PS-Cl microsphere prepared in this preparation example were shown in FIGs. 1 and 2. The PS-Cl microsphere was spherical with a smooth surface and has a diameter of 38-82 µm; and a strong absorption peak appeared near 2,930-2,850 cm⁻¹ and 700-750 cm⁻¹.

### Preparation Example 2

This preparation example was used for illustrating the preparation of a hydroxyl-modified polystyrene microsphere, which specifically included the following steps: 9.0 mL of styrene, 50 µL of divinylbenzene, 1 mL of 3-buten-1-ol, 22 mL of absolute ethanol and 19.5 mL of deionized water were accurately pipetted into a round-bottom flask, added with 1.0 g of the PS microsphere provided in the synthesis example and 3.0 g of polyethylene glycol 4000, and ultrasonically dispersed for 30 min; then added with 0.182 g of ammonium persulfate and stirred thoroughly, subjected to vacuum degassing and rapidly stirred until it became an emulsion; then reacted in an oil bath at 70°C under a nitrogen atmosphere for 12 h; and cooled to room temperature after the reaction was completed. The obtained emulsion was repeatedly washed with absolute ethanol for 10 times, then continually washed with deionized water for 8 times, and vacuum-dried at 60°C for 48 h to obtain a hydroxyl-modified polystyrene microsphere, which was recorded as a PS-OH microsphere.

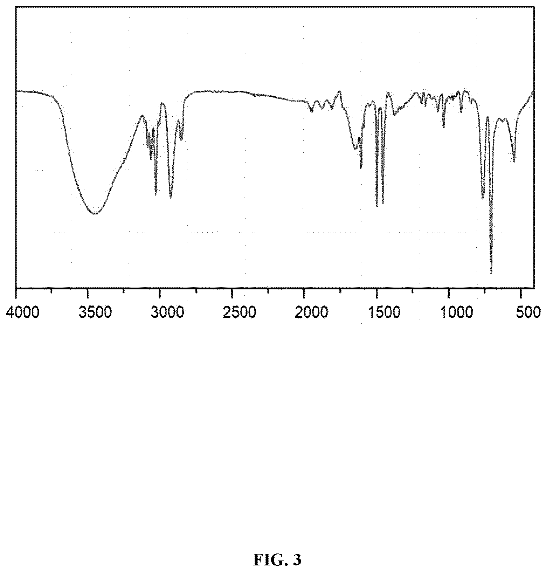

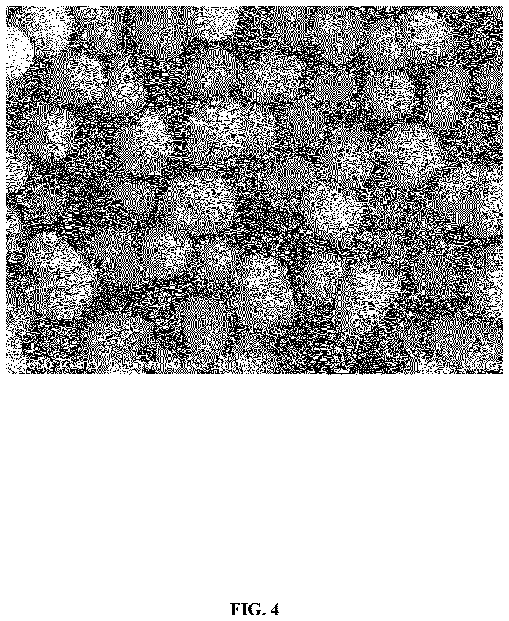

The SEM image and infrared spectrogram of the PS-OH microsphere prepared in this preparation example were shown in FIGs. 3 and 4. The PS-OH microsphere was spherical with certain defects on the surface thereof and has a diameter of 2-3.5 µm; and a strong absorption peak appeared near 3,650-3,600 cm⁻¹, 1,500-1,450 cm⁻¹ and 769-659 cm⁻¹.

### Preparation Example 3

This preparation example was used for illustrating the preparation of a carboxyl-modified polystyrene microsphere, which specifically included the following steps: 20 mL of styrene, 0.2 mL of divinylbenzene, 2.0 mL of linoleic acid and 75 mL of absolute ethanol were accurately pipetted into a round-bottom flask, added with 1.5 g of the PS microsphere provided in the synthesis example, 7.0 g of polyethylene glycol 4000 and 6.0 g of BPO, and ultrasonically dispersed for 30 min; subjected to vacuum degassing and rapidly stirred until it became an emulsion; then reacted in an oil bath at 70°C under a nitrogen atmosphere for 10 h; and cooled to room temperature after the reaction was completed. The obtained emulsion was repeatedly washed with absolute ethanol for 10 times, then washed with deionized water for 8 times, and vacuum-dried at 60°C for 48 h to obtain a carboxyl-modified polystyrene microsphere, which was recorded as a PS-COOH microsphere.

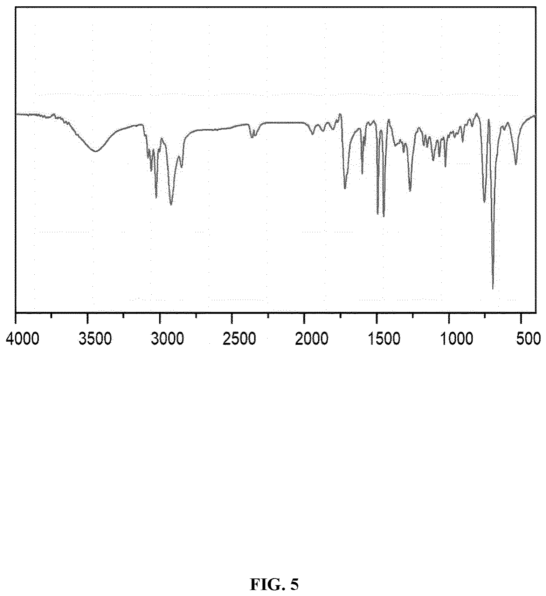

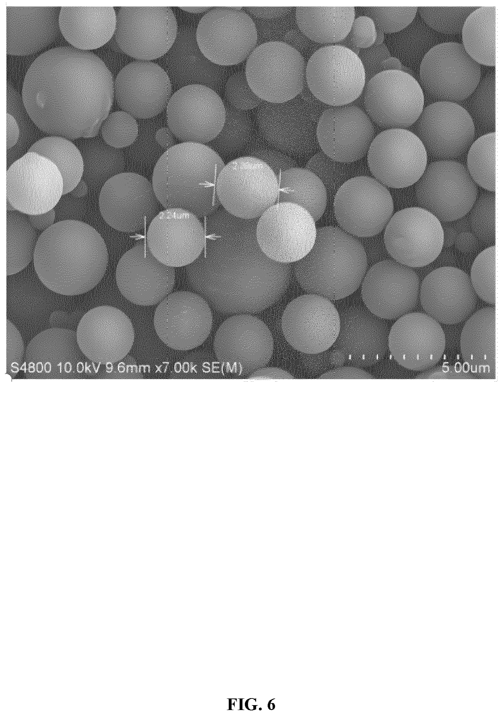

The SEM image and infrared spectrogram of the PS-COOH microsphere prepared in this preparation example were shown in FIGs. 5 and 6. The PS-COOH microsphere was spherical with a smooth surface and has a diameter of 2-3 µm; and a strong absorption peak appeared near 3,300-2,500 cm⁻¹, 1,720-1,210 cm⁻¹ and 750-720 cm⁻¹.

### Preparation Example 4

This preparation example was used for illustrating the preparation of a nitrogen-containing five-membered heterocyclic modified polystyrene microsphere, which specifically included the following steps: 9.0 mL of styrene, 15 mL of absolute ethanol and 125 mL of deionized water were accurately pipetted into a round-bottom flask, added with 652 mg of polyvinylpyrrolidone (PVP K-30, weight-average molecular weight of 58,000) and 122.5 mg of AIBN, subjected to vacuum degassing, and quickly stirred until it became an emulsion; then reacted in a 73°C oil bath under a nitrogen atmosphere for 15 h, then added with 1 mL of N-vinyl-2-pyrrolidone, continually reacted for 3 h; and cooled to room temperature after the reaction was completed. The obtained emulsion was repeatedly washed with absolute ethanol for 10 times, then continually washed with deionized water for 8 times, and vacuum-dried at 60°C for 48 h to obtain a nitrogen-containing five-membered heterocyclic modified polystyrene microsphere, which was recorded as a PS-N microsphere.

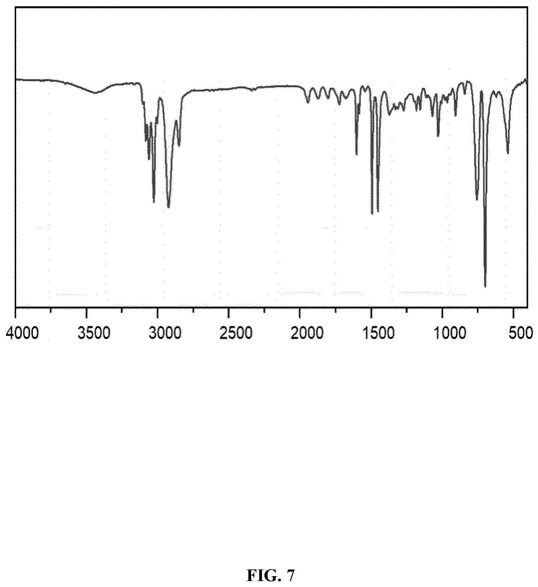

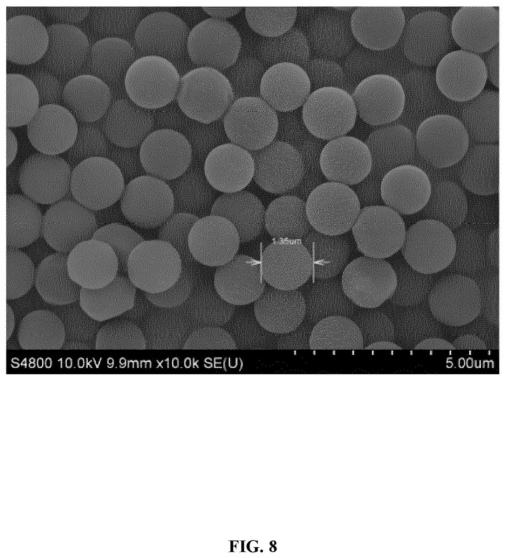

The SEM image and infrared spectrogram of the PS-N microsphere prepared in this preparation example were shown in FIGs. 7 and 8. The PS-N microsphere was spherical with a smooth surface and has a diameter of 1-1.5 µm; and a strong absorption peak appeared near 3,100-2,750 cm⁻¹,1,750-1,370 cm⁻¹ and 750-650 cm⁻¹.

### Example 1

This example is used for illustrating a method for preparing an RNA product, and the method specifically included the following steps:
S1. Pre-treatment: (1) 3.5 mg of the PS-Cl microsphere provided in Preparation Example 1 was taken and subjected to denuclease treatment by a centrifugal column method, repeatedly washed for 6 times with vortexing for 5 min each time, and centrifuged at 7,000 rpm for 30 s. The precipitate was added with RNase-free water to a final volume of 200 µL, so as to obtain an RNA room-temperature storage stabilizer. (2) In a sterile enzyme-free operating table, 10 µg of an IVT RNA was added into 200 µL of the RNA room-temperature storage stabilizer to obtain an RNA sample, which was mixed thoroughly and transferred into a vial, and the vial was half-stoppered.
S2. Freeze-drying: The half-stoppered vial was placed on a freeze-drying chamber shelf of a four-ring vacuum freeze dryer (LGJ-S30 capping type) at room temperature, and freeze-dried according to the procedure shown in Table 1 to obtain the RNA product.
S3. The vial containing the RNA product was transferred to a transition room of a glove box, evacuated and replaced with nitrogen, allowed to stand overnight and then transferred to an operating room, and the cap of the vial was sealed with wax.

The IVT RNA used in the step S1 of this example was synthesized by performing a PCR reaction with a pUC19 plasmid DNA as a template and primers of eGFP nucleotide sequences, and then subjecting to in vitro transcription with a T7 polymerase, and has a length of 850 nt.

**Table 1. Freeze-drying procedure**

| Stage | Temperature (°C) | Temperature change time (min) | Duration (h) | Target vacuum degree (Pa) |
|---|---|---|---|---|
| Pre-freezing stage | -50 | - | 10 | - |
| Primary drying stage | -40 | 30 | 3 | < 10 |
| Secondary drying stage | -30 | 30 | 3 | |
| | -20 | 30 | 3 | |
| | -10 | 30 | 3 | |
| | 0 | 30 | 3 | |
| | 10 | 30 | 3 | |
| | 25 | 30 | 35 | |

### Example 2

The preparation method of the RNA product provided in this example was basically the same as that in Example 1, except that in the step S1, the PS-Cl microsphere provided in Preparation Example 1 was replaced with equal mass of the PS-OH microsphere provided in Preparation Example 2, and the other conditions were the same, so as to obtain the RNA product.

### Example 3

The preparation method of the RNA product provided in this example was basically the same as that in Example 1, except that in the step S1, the PS-Cl microsphere provided in Preparation Example 1 was replaced with equal mass of the PS-COOH microsphere provided in Preparation Example 3, and the other conditions were the same, so as to obtain the RNA product.

### Example 4

The preparation method of the RNA product provided in this example was basically the same as that in Example 1, except that in the step S1, the PS-Cl microsphere provided in Preparation Example 1 was replaced with equal mass of the PS-N microsphere provided in Preparation Example 4, and the other conditions were the same, so as to obtain the RNA product.

### Example 5

The preparation method of the RNA product provided in this example was basically the same as that in Example 1, except that in the step S1, the PS-Cl microsphere provided in Preparation Example 1 was replaced with equal mass of the epoxy-modified polystyrene microsphere (available from Suzhou Knowledge & Benefit Sphere Tech. Co., Ltd., with a brand name of KBsphere^{®} PSEP), and the other conditions were the same, so as to obtain the RNA product.

### Example 6

The preparation method of the RNA product provided in this example was basically the same as that in Example 1, except that in the step S1, the addition amount of the IVT RNA was 1 µg, and the other conditions were the same, so as to obtain an RNA product.

### Example 7

The preparation method of the RNA product provided in this example was basically the same as that in Example 1, except that in the step S1, the addition amount of the IVT RNA was 200 µg, and the other conditions were the same, so as to obtain an RNA product.

### Example 8

The preparation method of the RNA product provided in this example was basically the same as that in Example 1, except that in the step S2, the freeze-drying conditions were different, specifically as shown in Table 2, so as to obtain an RNA product.

**Table 2. Freeze-drying procedure**

| Stage | Temperature (°C) | Temperature change time (min) | Duration (h) | Target vacuum degree (Pa) |
|---|---|---|---|---|
| Pre-freezing stage | -50 | - | 10 | - |
| Primary drying stage | -40 | 10 | 3 | < 10 |
| Secondary drying stage | -30 | 10 | 3 | |
| | -20 | 10 | 3 | |
| | -10 | 10 | 3 | |
| | 0 | 10 | 3 | |
| | 10 | 10 | 3 | |
| | 25 | 10 | 35 | |

### Example 9

The preparation method of the RNA product provided in this example was basically the same as that in Example 1, except that in the step S2, the half-stoppered vial was directly placed at -70°C for freeze drying for 63 h, and the other conditions were the same, so as to obtain an RNA product.

### Comparative Example

This comparative example provided a method for preparing an RNA product, which specifically included the following steps:
S1. Pre-treatment: 10 µg of an IVT RNA was added into 200 µL of RNase-free water to obtain an RNA sample, which was mixed thoroughly and then transferred into a vial, and the vial was half-stoppered.
S2. Freeze-drying: The half-stoppered vial was placed on a freeze-drying chamber shelf of a four-ring vacuum freeze dryer (LGJ-S30 capping type) at room temperature, and freeze-dried according to the same procedure as that in Example 1 (i.e., Table 1) to obtain the RNA product.
S3. The vial containing the RNA product was transferred to a transition room of a glove box, evacuated and replaced with nitrogen, allowed to stand overnight and then transferred to an operating room, and the cap of the vial was sealed with wax.

### Test Example

This test example was used for illustrating the storage stability of the RNA products provided in the aforementioned examples and comparative examples. The RNA products were stored at 25°C, 37°C and 60°C for a period of time and then taken out. In a sterile enzyme-free operating table, 100 µL of RNase-free water was taken to rehydrate the RNA product, and the mixture was shaken and mixed evenly for 15 min. The mixture was centrifuged at 7,000 rpm for 4 min, and the supernatant was taken and filtered with a 0.22 µm RNase-free filter membrane. An RNA product added with trehalose added as a protective agent was used as a positive control group. The preparation of the RNA product specifically included the following steps: in a sterile enzyme-free operating table, 10 µg of an IVT RNA was added into 200 µL of a solution of trehalose in RNase-free water with a concentration of 1.72% (w:v) to obtain an RNA sample, which was mixed thoroughly and then transferred into a vial, and the vial was half-stoppered; the half-stoppered vial was placed on a freeze-drying chamber shelf of a four-ring vacuum freeze dryer (LGJ-S30 capping type) at room temperature, and freeze-dried according to the same procedure as that in Example 1 (i.e., Table 1) to obtain the RNA product. Detection was performed according to the following method:
(1) Detection by capillary electrophoresis: 1 µL of the supernatant was taken and detected with an automatic capillary electrophoresis instrument (Agilent Fragment Analyzer 5200) to obtain the percentage of a normal IVT RNA concentration to a total RNA concentration. The results were shown in Table 3.

**Table 3. Storage stability of RNA products**

| Groups | Percentage of Normal RNA concentration to total RNA concentration (%) | | |
|---|---|---|---|
| Storage temperature | 25°C | 37°C | 60°C |
| Storage time | 21 d | 21 d | 14 d |
| Positive control group | 88.3 | 85.0 | 55.4 |
| Example 1 | 86.5 | 80.3 | 57.2 |
| Example 2 | 76.6 | 71.1 | 47.1 |
| Example 3 | 73.6 | 68.6 | 58.3 |
| Example 4 | 78.5 | 72.2 | 48.9 |
| Example 5 | 69.6 | 40.3 | 19.8 |
| Example 6 | 88.4 | 85.3 | 55.9 |
| Example 7 | 75.2 | 65.3 | 36.4 |
| Example 8 | 75.0 | 74.8 | 43.1 |
| Example 9 | 60.1 | 50.0 | 40.6 |
| Comparative Example | 46.4 | 35.5 | - |

| | | | |
|---|---|---|---|
| Note: -- indicated that normal RNA bands could no longer be detected and were completely degraded | | | |

It could be seen from the test results that, compared with the comparative example, the RNA products provided by Examples 1-9 of the present disclosure and the positive control group could still maintain good RNA integrity after being stored at 37°C for 21 d.

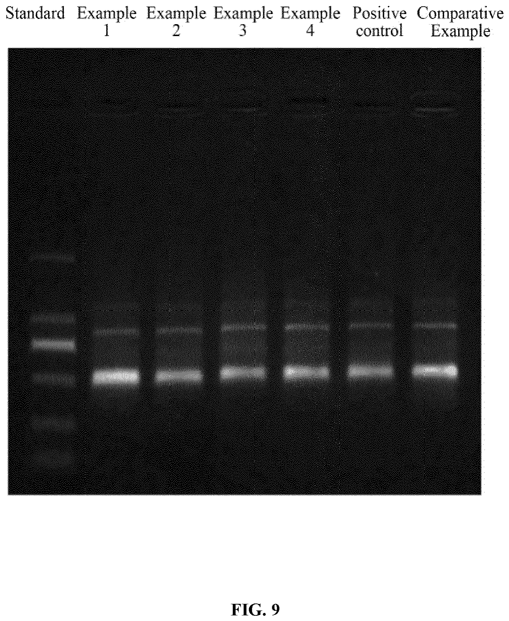

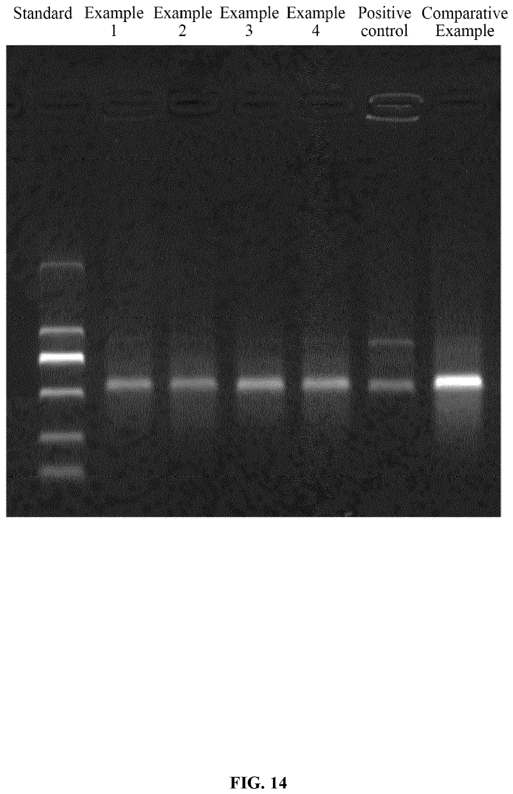

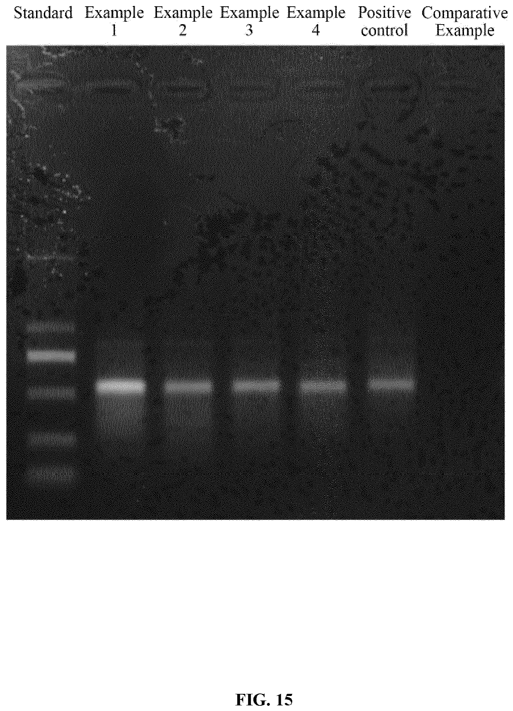

(2) Detection with agarose gel electrophoresis: (i) 0.5 g of agarose was taken and mixed with 50 mL of a 1 × TAE solution, and heated over medium heat in a microwave oven until completely dissolved to obtain a 1% agarose gel, which was cooled for several seconds and then poured into an electrophoresis mold, and cooled into a gel. The comb was removed, and the gel was placed into a TAE electrophoresis tank, and the 1 × TAE solution was poured into the TAE electrophoresis tank, so that the wells of the gel were filled up with liquid and the solution submerged the agarose gel.
(ii) 500 mg of sucrose, 500 µL of water and 5 µL of 10,000 × GeL Red were taken to obtain a Loading Dye loading buffer; 3 µL of Ladder (TaKaRa, catalog number 3427A) was taken, added with 15 µL of RNase-free water, and then added with 2 µL of the Loading Dye loading buffer to obtain a Ladder electrophoresis loading solution.
(iii) 500 ng of the supernatant and 2 µL of the Loading Dye loading buffer were taken, and added with RNase-free water to a final volume of 20 µL, so as to obtain an RNA electrophoresis loading solution.
(iv) The Ladder electrophoresis loading solution and the RNA electrophoresis loading solution were taken and added into the wells of the agarose gel in the electrophoresis tank at once, covered with a lid, and subjected to electrophoresis at 120 V for 30 min; and then the gel was transferred to a Bio-Rad instrument and photographed. The results were shown in FIGs. 9-15.

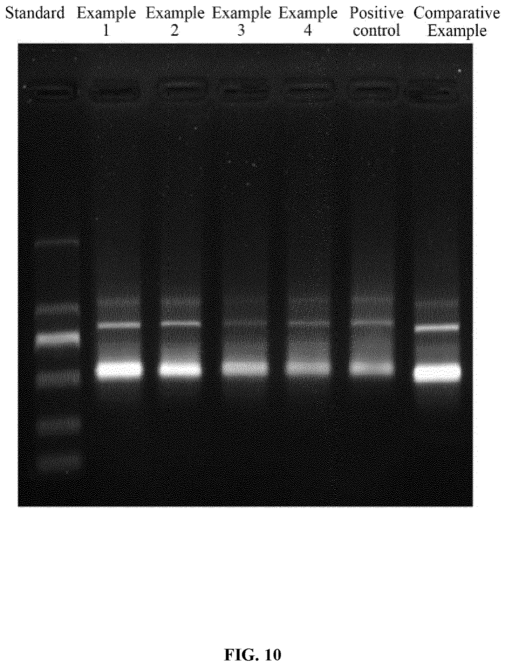

It could be seen from FIGs. 9 and 10 that, after being stored at 25°C for 7 d and 21 d, the bands of the Comparative Example showed slight downward diffusion, indicating that part of the RNA was degraded, while the bands of Examples 1-4 of the present disclosure were clearly visible and did not diffuse downward, indicating that the RNA room-temperature storage stabilizer had a good protective and stabilizing effect on the freeze-drying of the RNA and the storage process of the RNA at 25°C.

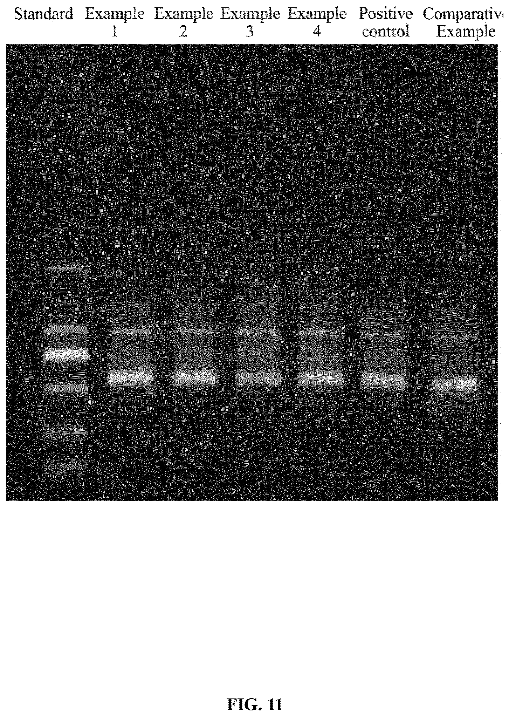

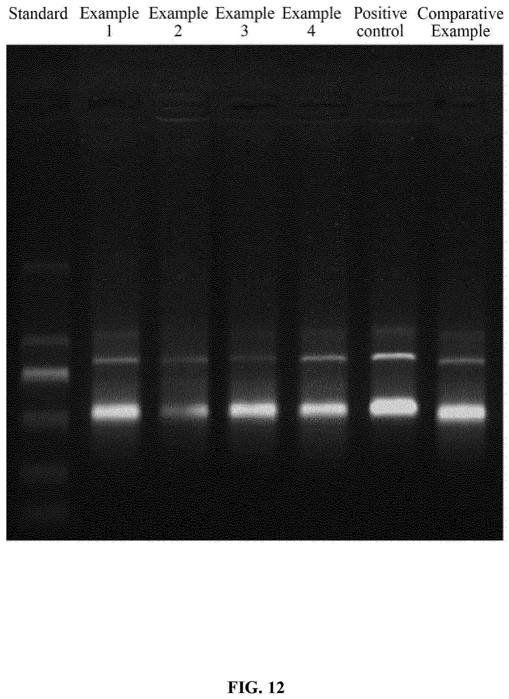

It could be seen from FIGs. 11 and 12 that, after being stored at 37°C for 7 d and 21 d, the bands of the Comparative Example showed slight downward diffusion, indicating that part of the RNA was degraded, while the bands of Examples 1-4 of the present disclosure were clearly visible and did not diffuse downward, indicating that the RNA room-temperature storage stabilizer had a good protective and stabilizing effect on the freeze-drying of the RNA and the storage process of the RNA at 37°C.

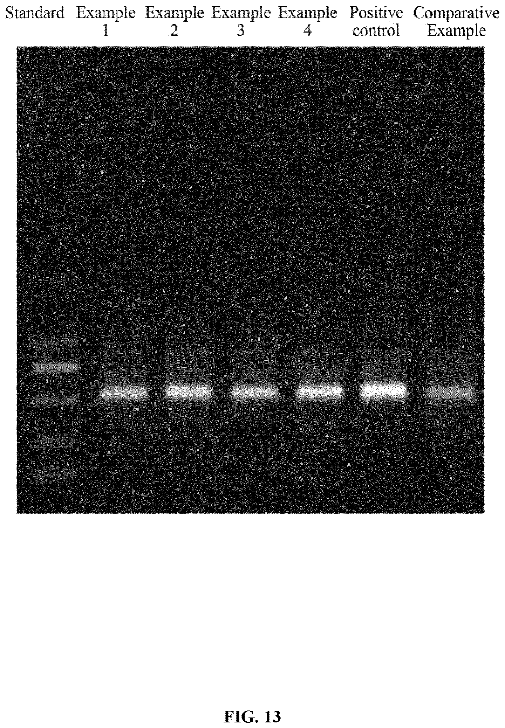

It could be seen from FIGs. 13-15, after being stored at 60°C for 1 d and 5 d, the bands of the Comparative Example showed slight and obvious downward diffusion, respectively, indicating that the RNA was degraded at 5 d, while the bands of Examples 1-4 of the present disclosure were not significantly degraded; and after being stored at 60°C for 14 d, the bands of the Comparative Example completely disappeared, while the bands of Examples 1-4 of the present disclosure were still clearly visible, with only partial degradation occurred, indicating that the RNA room-temperature storage stabilizer had a good protective and stabilizing effect on the freeze-drying of the RNA and the storage process of the RNA at 60°C.

Although the embodiments of the present disclosure have been shown and described above, it is to be understood that the aforementioned embodiments are illustrative and are not to be construed as limitations on the present disclosure. Changes, modifications, substitutions and variations can be made to the aforementioned embodiments within the scope of the present disclosure by those of ordinary skills in the art, without departing from the principle and spirit of the present disclosure.

## Claims

1. An RNA room-temperature storage stabilizer, comprising a functionalized polystyrene microsphere.

2. The RNA room-temperature storage stabilizer according to claim 1, wherein the functionalized polystyrene microsphere is a polystyrene microsphere modified with a functional group, and the functional group is selected from one or more of C1-C20 chloroalkyl, C6-C20 chlorophenyl, C1-C20 hydroxyl-substituted alkyl, C6-C20 hydroxyl-substituted phenyl, C2-C20 carboxyl-substituted alkyl, C7-C20 carboxyl-substituted phenyl, C1-C20 amino-substituted alkyl, C6-C20 amino-substituted phenyl, and C4-C20 nitrogen-containing heterocycle.

3. The RNA room-temperature storage stabilizer according to claim 1, wherein the functionalized polystyrene microsphere is selected from one or more of a chloromethyl modified polystyrene microsphere, a hydroxyl modified polystyrene microsphere, a carboxyl modified polystyrene microsphere, and a nitrogen-containing five-membered heterocyclic modified polystyrene microsphere.

4. The RNA room-temperature storage stabilizer according to claim 1, wherein an average particle size of the functionalized polystyrene microsphere is 0.1-200 µm.

5. The RNA room-temperature storage stabilizer according to claim 1, wherein the average particle size of the functionalized polystyrene microsphere is 1-2 µm.

6. A method for preparing the RNA room-temperature storage stabilizer according to claim 1, comprising: step S1: taking a polymerizable monomer, a functional modifier, an initiator and an optional polystyrene particle to conduct a polymerization reaction to obtain the functionalized polystyrene microsphere; and step S2: taking and subjecting the functionalized polystyrene microsphere to denuclease treatment to obtain the RNA room-temperature storage stabilizer.

7. The method for preparing the RNA room-temperature storage stabilizer according to claim 6, wherein in the step S1, the polymerizable monomer is selected from one or more of styrene, phenylpropene and divinylbenzene.

8. The method for preparing the RNA room-temperature storage stabilizer according to claim 6, wherein in the step S1, the functional modifier is selected from one or more of a chlorinated olefin, an enol, an unsaturated fatty acid and an olefinized pyrrolidone.

9. The method for preparing the RNA room-temperature storage stabilizer according to claim 6, wherein in the step S1, the initiator is an azo initiator and/or a peroxy initiator.

10. The method for preparing the RNA room-temperature storage stabilizer according to claim 6, wherein in the step S1, an average particle size of the polystyrene particle is 0.01-1 µm.

11. The method for preparing the RNA room-temperature storage stabilizer according to claim 6, wherein in the step S1, an input mass ratio of the polymerizable monomer, the functional modifier, the initiator and the polystyrene particle is (5-20):(0.1-20):(0.1-10):(0.001-20).

12. The method for preparing the RNA room-temperature storage stabilizer according to claim 6, wherein in the step S1, the polymerization reaction is conducted at a temperature of 70-90°C for a time of 1-48 h.

13. The method for preparing the RNA room-temperature storage stabilizer according to claim 6, wherein in the step S2, the denuclease treatment specifically comprises: taking and mixing the functionalized polystyrene microsphere with RNase-free water, and then subjecting to centrifugal column purification to obtain the RNA room-temperature storage stabilizer.

14. The method for preparing the RNA room-temperature storage stabilizer according to claim 13, wherein in the step S2, the centrifugal column purification is conducted at a centrifugal speed of 5,000-1,0000 rpm for a time of 5-30 min.

15. An RNA product, comprising the RNA room-temperature storage stabilizer according to claim 1.

16. A method for preparing the RNA product according to claim 15, comprising: taking and mixing an RNA with the RNA room-temperature storage stabilizer, and then subjecting to freeze-drying treatment to obtain the RNA product.

17. The method for preparing the RNA product according to claim 16, wherein an input mass ratio of the RNA to the RNA room-temperature storage stabilizer is 1:(10-1000).

18. The method for preparing the RNA product according to claim 16, wherein the freeze-drying treatment comprises a pre-freezing stage, a primary drying stage and a secondary drying stage, the pre-freezing stage is conducted at a temperature of -60°C to -50°C for a time of 5-15 h; the primary drying stage is conducted at a temperature of -45°C to -40°C with a vacuum degree no higher than 10 Pa for a time of 1-5 h; the secondary drying stage comprises a heating period and a constant temperature period that are conducted alternately, the secondary drying stage is conducted at a starting temperature of -35 - -30°C and a final temperature of 20-25°C with a vacuum degree no higher than 10 Pa for a total time of 40-60 h, and the heating period is conducted at a heating rate of 0.5-2°C/min for a time of 25-40 min.

19. Use of the RNA room-temperature storage stabilizer according to claim 1 in production of a mRNA drug and a mRNA vaccine.
